# EUROPEAN PATENT SPECIFICATION

(11) **EP 1 379 557 B1**
(45) Date of publication and mention of the grant of the patent: **17.09.2008**
(21) Application number: 02740446.6
(22) Date of filing: 05.04.2002
(51) Int. Cl.: C07K 16/28

(54) **USE OF CD25 BINDING MOLECULES IN STEROID-RESISTANT PATIENTS**
VERWENDUNG VON CD25-BINDENDER MOLEKÜLE FÜR PATIENTEN MIT STEROIDE-RESISTENZ
UTILISATION DE MOLECULES DE LIAISON CD25 CHEZ DES PATIENTS RESISTANTS AUX STEROIDES

(30) Priority: 06.04.2001 GB 0108821; 06.04.2001 GB 0108817; 06.04.2001 GB 0108816
(43) Date of publication of application: 14.01.2004
(73) Proprietor: UNIVERSITY OF BRISTOL, Bristol BS8 1TH (GB)
(72) Inventor: HEARING, Stephen David, Stone, Staffordshire ST15 8ZS (GB); DAYAN, Colin Mark, Bristol BS2 8BX (GB); NORMAN, Michael Roden, Bristol BS6 5SX (GB)
(74) Representative: Nash, David Allan
(86) International application number: PCT/EP2002/003808
(87) International publication number: WO 2002/081508

(56) References cited:
- EP-A- 0 449 769
- WO-A-00/06604
- WO-A-00/25816
- WO-A-01/72845
- WO-A-90/07861
- QUEEN C ET AL: "A HUMANIZED ANTIBODY THAT BINDS TO THE INTERLEUKIN 2 RECEPTOR" PROCEEDINGS OF THE NATIONAL ACADEMY OF SCIENCES OF USA, NATIONAL ACADEMY OF SCIENCE. WASHINGTON, US, vol. 86, no. 24, 1 December 1989 (1989-12-01), pages 10029-10033, XP000310534 ISSN: 0027-8424
- KOVARIK J ET AL: "DISPOSITION AND IMMUNODYNAMICS OF BASILIXIMAB IN LIVER ALLOGRAFT RECIPIENTS" CLINICAL PHARMACOLOGY & THERAPEUTICS, MOSBY-YEAR BOOK, ST LOUIS, MO, US, vol. 64, no. 1, July 1998 (1998-07), pages 66-72, XP000876964 ISSN: 0009-9236
- HOGEZAND VAN R A ET AL: "SELECTIVE IMMUNOMODULATION IN PATIENTS WITH INFLAMMATORY BOWEL DISEASE-FUTURE THERAPY OR REALITY?" NETHERLANDS JOURNAL OF MEDICINE, ELSEVIER SCIENCE BV., AMSTERDAM, NL, vol. 48, no. 2, 1996, pages 64-67, XP001008578 ISSN: 0300-2977
- VOIGLIO E ET AL: "ACTIVATION DES LYMPHOCYTES T DANS LA MALADIE DE CROHN ET DANS LA RECTOCOLITE ULCERO-HEMORRAGIQUE IMPLICATIONS THERAPEUTIQUES T-LYMPHOCYTES ACTIVATION IN CROHN'S DISEASE AND IN ULCERATIVE COLITIS. THERAPEUTICAL IMPLYINGS" PATHOLOGIE BIOLOGIE, L'EXPANSION SCIENTIFIQUE FRANCAISE, PARIS, FR, vol. 44, no. 4, April 1996 (1996-04), pages 287-292, XP001008579 ISSN: 0031-3009

### SEQUENCE LISTING

<110> University of Bristol
   Hearing, Stephen
   Dayan, Colin
   Norman, Michael
<120> use of CD25 binding molecules in steroid-resistant patients
<130> 4-32296A/UOB
<140> PCT/EP02/03808
   <141> 2002-04-05
<150> GB 0108821.0
   <151> 2001-04-06
<150> GB 0108817.8
   <151> 2001-04-06
<150> GB 0108816.0
   <151> 2001-04-06
<160> 6
<170> PatentIn version 3.0
<210> 1
   <211> 5
   <212> PRT
   <213> Artificial/Unknown
<220>
   <221> misc_feature
   <222> ()..()
   <223> CDR sequence
<400> 1
   Arg Tyr Trp Met His
   1               5
<210> 2
   <211> 17
   <212> PRT
   <213> Artificial/Unknown
<220>
   <221> misc_feature
   <222> ()..()
   <223> CDR sequence
<400> 2
   Ala Ile Tyr Pro Gly Asn Ser Asp Thr Ser Tyr Asn Gln Lys Phe Glu
   1               5                   10                  15
   Gly
<210> 3
   <211> 8
   <212> PRT
   <213> Artificial/unknown
<220>
   <221> misc_feature
   <222> ()..()
   <223> CDR sequence
<400> 3
   Asp Tyr Gly Tyr Tyr Phe Asp Phe
   1               5
<210> 4
   <211> 10
   <212> PRT
   <213> Artificial/unknown
<220>
   <221> misc_feature
   <222> ()..()
   <223> CDR sequence
<400> 4
   Ser Ala Ser Ser Ser Ile Ser Tyr Met Gln
   1               5                   10
<210> 5
   <211> 7
   <212> PRT
   <213> Artificial/Unknown
<220>
   <221> misc_feature
   <222> ()..()
   <223> CDR sequence
<400> 5
   Asp Thr Ser Lys Leu Ala Ser
   1               5
<210> 6
   <211> 7
   <212> PRT
   <213> Artificial/unknown
<220>
   <221> misc_feature
   <222> ()..()
   <223> CDR sequence
<400> 6
   His Gln Arg Ser Ser Tyr Thr
   1               5

## Description

The present invention relates to the CD25 binding molecules for use in the treatment of a disease selected from the group consisting of autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease in steroid-resistant patients.

### Ulcerative Colitis

For most patients suffering from ulcerative colitis, steroids are an effective treatment. However, between 30 % and 50 % of patients with ulcerative colitis do not respond to steroid therapy and medical therapy of these patients remains unsatisfactory and many require surgery. Prior to the introduction of steroids as a treatment for severe ulcerative colitis, the mortality rate was of the order of 50% (Truelove SC, & Witts LJ. BMJ 1955; ii:1041-8). The current practice is to offer those patients with severe ulcerative colitis, not responding to 5 to 7 days steroid therapy, urgent surgery (Jewell DP, et al. Gastroenterol Int 1991; 4:161-4). Surgery, although curative involves an ileostomy, at least temporarily, and thus patients are often reluctant to undergo surgery. However, delaying surgery in patients who have not responded to medical therapy is dangerous as preoperative perforation can occur which is associated with an increased mortality (Ritchie JK. BMJ 1974; i:264-268). As an alternative to surgery, the immunosuppressive drug cyclosporine has been used to treat some patients with steroid-resistant ulcerative colitis (Lichtiger S et al. N Engl J Med 1994; 330:1841-5). However the long term results are disappointing with many patients who respond initially requiring surgery within six months and many patients experiencing side-effects including fatal opportunistic infections such as *Pnemocystis carinii* pneumonia, due to immonusupression, renal failure and hypertension (Smith MB, N Engl J Med 1992; 327:497-8; Sandborn WJ. Inflamm Bowel Dis 1995;1:48-63). Azathioprine is an effective therapy for chronically active, steroid-resistant or steroid-dependant ulcerative colitis. However, its delayed onset of action (3-4 months) means it is not suitable for patients with severe ulcerative colitis who require urgent treatment (Ardixxone S et al. J Clin Gastroenterol 1997; 25:330-3.) Thus, treatment of steroid-resistant ulcerative colitis remains difficult and usually requires surgery resulting in a stoma. Alternative medical treatment of steroid-resistant ulcerative colitis is needed, and identifying this requires an understanding of the reasons why some patients with ulcerative colitis fail to respond to steroid therapy. For an individual patient it is difficult to predict the response to steroid therapy and thus, the requirement for surgery or alternative medical therapy. In severe ulcerative colitis, initial measures of disease severity correlate poorly with the eventual outcome (response to steroid therapy). (Lindgren SC, et al Eur J Gastrenterol Hepatol 1998; 10:831-5). Thus, the patents who fail to respond to steroid therapy are not necessarily the patients with the most severe disease.

### Asthma

The current treatment for asthma is the administration of steroids such as glucocorticoids. However, despite the usual favorable response, there are a group of patients who do not respond to steroid-therapy. Such patients are referred to as having "steroid-resistant asthma", and were first identified in 1968. Subsequently, steroid-resistant asthma has been defined as the failure to improve forced expiratory volume (FEV₁; a measure of lung function which is reduced in asthma) in 1 second by 15% after 7 days of prednisolone of a dosage of 20 mg per day (Carmichael J, et al, BMJ 1981; 282:1419-22*)*. Patients with steroid-resistant asthma have been compared to steroid-sensitive asthmatics, and baseline pulmonary function test results and the response to bronchodilators are not significantly different, indicating that steroid-resistant asthmatics do not have more severe disease. Lymphocyte steroid sensitivity of peripheral blood lymphocytes from patients with steroid-resistant asthma has been measured using inhibition of phytohaemagglutinin (PHA) stimulated proliferation by glucocorticoids (Poznansky MC, et al, Clin Sci 1984; 67:639-45)(Alvarez J, et al, J Allergy Clin Immunol 1992; 89:714-21*)*(Corrigan CJ, et al, Am Rev Respir Dis 1991; 144:1026-32)(Hackzu A, et al, J Allergy Clin Immunol 1994; 93:510-9)(Spahn JD, et al, J Allergy Clin Immunol 1996; 98:1073-9*)*. All studies showed that lymphocytes from steroid-resistant asthmatics were inhibited significantly less by steroids than lymphocytes from steroid-sensitive asthmatics indicating that the patients with steroid-resistant asthma had steroid-resistant lymphocytes. In a further study, 8 patients with steroid-resistant asthma, were found to have steroid-resistant lymphocytes and were treated with methotrexate (Vrugt B, et al, Eur Respir J 2000; 15:478-85*)*. After 8 weeks bronchial muscosal inflammation was reduced and lymphocyte steroid sensitivity had increased. It was argued that the beneficial effect of methotrexate on the bronchial muscosa in steroid-resistant asthma was due to increasing steroid sensitivity. This is an example of a drug acting as a "steroid enhancer" and increasing both lymphocyte steroid sensitivity and being clinically effective. Approximately 25% of patients with "difficult to treat" asthma, referred to a tertiary referral centre were found to have steroid-resistant lymphocytes (Chan MTS, et al, J Allergy Clin Immunol 1998; 101:594-601*)*.

### Autoimmune hepatitis

Autoimmune hepatitis is a form of chronic liver disease characterized by progressive hepatocellular inflammation, which usually responds to treatment with corticosteroids. However, 10% of patients with autoimmune hepatitis are refractory to corticosteroids and develop progressive liver disease and cirrhosis. Treatment of this group of patients with steroid-resistant disease is difficult and can require transplantation. Cyclosporin has been used as a treatment for steroid-resistant autoimmune hepatitis (Femandes NF et al, Am J Gastroenterol 1999; 94: 241-8*).* In a trial of 5 patients this was successful in 4 patients. Unfortunately, the single patient who did not respond to cyclosporin developed progressive liver failure, underwent orthotopic liver transplantation, and subsequently died of disseminated cytomegalovirus infection. Cyclosporin was generally well tolerated and none of the patients developed renal insufficiency. It would be expected that the patients with steroid-resistant autoimmune hepatitis have steroid-resistant lymphocytes.

### Eczema

Eczema is a distinctive pattern of skin inflammation which can be induced or maintained by a variety of environmental or intrinsic factors, e.g. contact allergen and irritants, infective agents and atopy. The majority of patients with eczema have mild disease and require intermittent topical therapy. However some patients have more severe disease and require systemic steroid therapy (Hoare C, et al, Health Technol Assess 2000; 4: 1-191*)*(Heddle RJ, et al, Br Med J (Clin Res Ed) 1984; 289: 651-4*)*. Failure to respond to steroid therapy requires alternative therapy with immunosuppressive drugs such as cyclosporin (Granlund H, et al Acta Derm. Venereol. 1996; 76: 371-6*)*. This failure to respond to steroid therapy is expected to be due to the steroid-resistant patient having steroid- resistant lymphocytes.

### Vasculitis

The vasculitides are a group of conditions characterized by inflammation of the arteries. Some of these conditions also cause glomerulonephritis (including Wegner's granulomatosis, Goodpasture's syndrome and polyarteritis nodosa). Steroids remain first line treatment for all these conditions (Bosch X, et al Lupus 1999; 8:258-62*)*(Guillevin L. nephrol.Dial.TRansplant 199;14:2077-9*)*(Reinhold-Keller E, et al Arthritis Rheum. 2000; 43:1021-32*).* Immunosuppressive therapy is usually given with steroids, and failure of this combined therapy can be fatal. It is thought that a substantial number of the patients who fail to respond to treatment will have steroid-resistant lymphocytes.

### Temporal arteritis

Temporal arteritis is also a form of vasculitis. A complication is blindness due to interference with the blood supply to the eye caused by the inflammation in the artery. Approximately 15% of patients do not respond to first line steroid treatment (Chevalet P. et al, J Rheumatol. 2000; 27:1484-91*)*. These patients are often treated with prolonged, ineffective courses of steroids. Alternative therapies have been used such as methotrexate for steroid-resistant disease (Krall PL, et al, Cleve Clin J Med 1989; 56:253-7*)*. This failure to respond to steroid therapy is expected to be due to the steroid-resistant patient having steroid-resistant lymphocytes.

### Sarcoid

Sarcoidosis is a systemic granulomatous disorder of unknown cause. Sarcoidosis results from an overexuberant T cell-mediated immune response to the unknown antigen. Steroids remain the first-line therapy (Barnard J, et al, Curr Opin Rheumatol. 2001; 13:84-91*).* Alternatives to steroids are often introduced either because of steroid intolerance or in an attempt to reduce steroid dose and side effects.

### Systemic lupus erythematosis

Systemic lupus erythematosis is an autoimmune disorder that includes abnormalities in T lymphocytes, as well as hyper-reactive B cells that produce auto-antibodies. Left untreated the disease can result in end-stage renal disease or death.
Glucocorticoids are the drugs of first choice for the treatment of systemic lupus erythematosis. However, some patients fail to respond to steroid therapy.
The correlation between in vitro lymphocyte steroid sensitivity and clinical response to steroid therapy has also been examined in patients with systemic lupus erythematosis (SLE) (Seki M, et al, Arthritis Rheum 1998; 41:823-30*)*. Twenty-seven Japanese women treated with oral prednisolone (50-60 mg per day) for 1 month were studied. Nineteen patients were classified as steroid-sensitive and 8 as steroid-resistant according to clinical response to steroid treatment. Lymphocyte steroid sensitivity was determined by measuring in vitro steroid induced apoptosis (cell death) of peripheral blood lymphocytes.
Lymphocytes from SLE patients in the steroid-resistant group had a significantly lower percentage of apoptotic cells compared to the steroid-sensitive group at high concentrations of prednisolone (44.8% vs. 68.8%; p <0.05). Thus, although the method of measuring lymphocyte steroid sensitivity was different to the method which measures the level of inhibition of PHA stimulated lymphocyte proliferation by glucocorticoids, the patients who did not respond to steroid therapy had steroid-resistant lymphocytes.

### Leukaemia

Leukaemia is a malignant disorder of white blood cells in which the white blood cell precursors proliferate and fail to differentiate. Although leukaemia is not an inflammatory condition, glucocorticoids are included in the chemotherapy regimes used to treat this condition. Glucocorticoids induce lymphoblast apoptosis and this has been used as a measure of steroid sensitivity in acute lymphoblastic leukaemia (ALL). A high rate of in vitro apoptosis is associated with a good clinical response. Conversely steroid-resistance occurs in approximately 30% of newly diagnosed cases of ALL and represents a significant clinical problem (Kaspers GJL, et al, Leuk Lymphoma 1994;13:187-201*)*. Two studies have compared in vitro lymphocyte sensitivity, not only to steroids (dexamethasone and prednisolone), but also to L-asparginase and vincristine (Kaspers GLJ, et al, Blood 1997; 90:2723-9*)*(Hongo T, et al, Blood 1997; 89:2959-65). Using lymphocyte sensitivity to these drugs as a measure, patients were classified as sensitive (sensitive to all drugs), intermediate (sensitive to two or three drugs), or resistant (sensitive to one or no drugs). Three year disease free survival was highest in sensitive patients (85-100%) and lowest in resistant patients (43-55%). Thus lymphoblastic in vitro sensitivity and in vivo disease sensitivity in patients with ALL correlates not only for steroid sensitivity but also for other chemotherapeutic agents.

### Glomerulonephritis

Glomerulonephritis is characterized by inflammation of the kidney and is usually autoimmune. Steroids are an important treatment (Austin HA, et al, N Engl J Med 1986; 314:614-9*),* and failure of steroid therapy for this condition can lead to renal failure. Failure of steroid treatment in patients with steroid-resistant renal disease has lead to trials of immunosuppressive drugs (as in ulcerative colitis)(McCauley J, et al, Nephrol Dial Transplant 1993; 8:1286-90*).* A study has shown a range of lymphocyte steroid sensitivity in 16 patients with glomerulonephritis (Briggs WA, et al, J Clin Pharmacol 1996; 36:707-14*)*. It was argued that the range of lymphocyte steroid sensitivity explained the variable response to steroid therapy, however, in this study there was no comparison of lymphocyte steroid sensitivity with clinical disease response. Focal segmental glomerulosclerosis (FSGS) is a form of glomerulonephritis, which is also treated with steroids, although some patients do not respond (Ingulli E, et al, J Am Soc Nephrol 1995; 5:1820-5*)*. In this condition, lymphocyte steroid sensitivity has been compared to clinical responses and was shown to predict improvement in renal function in response to steroid treatment. Patients with the most sensitive lymphocytes responded best to steroid therapy, whilst those patients with steroid-resistant lymphocytes responded poorly to steroids.

### Multiple Sclerosis

Multiple sclerosis is an inflammatory demyelinating disease of the central nervous system in which T lymphocytes reactive with myelin proteins play an important pathogenic role. Glucocorticoids are used to treat multiple sclerosis (Brusaferri F, et al, J Neurol. 2000; 247:435-42*)*. Steroid treatment is effective in accelerating short-term recovery in patients with multiple sclerosis and may also be effective in reducing the risk of relapse. However, the clinical response varies widely amongst different patients. Lymphocyte steroid sensitivity has been measured in patients with multiple sclerosis (Wei T, et al, Acta Neurologica Scandinavica 1997; 96:28-33*)* and a wide range of lymphocyte sensitivity was found. No study has compared lymphocyte steroid sensitivity with clinical response of multiple sclerosis to steroid therapy but it has been argued that the range of in vitro lymphocyte steroid sensitivity may partly explain why response to steroid therapy is variable.

### Lymphocyte Steroid Sensitivity

As mentioned above, lymphocytes can be stimulated to proliferate *in vitro* by the mitogen PHA (Lindahl Kiessling K, Peterson RD. Exp Cell Res 1969; 5585-7). Addition of steroids to the culture medium inhibits lymphocyte proliferation (Walker KB et al Transplant Proc 1985; 17:1676-8). In some cases lymphocytes are steroid-sensitive with the addition of a small amount of steroid producing marked inhibition of proliferation and in other cases lymphocytes are steroid-resistant with addition of even large amounts of steroid producing minimal inhibition of proliferation. The inventors have previously studied the association between LSS and clinical outcome, in response to steroid therapy, in a group of patients with severe ulcerative colitis. (Hearing SD et al Gut 1999; 45:382-8). All patients were treated with a standard dose of intravenous steroids and the lymphocyte steroid sensitivity (LSS) of the patients was measured using dexamethasone inhibition of PHA induced proliferation of peripheral blood lymphocytes. A significant correlation between clinical outcome and LSS was demonstrated. All those patients with steroid-sensitive lymphocytes had a complete response to steroid therapy and all those patients with steroid-resistant lymphocytes had a poor response to steroid therapy. Thus, in ulcerative colitis there is a correlation between LSS and clinical response to steroid therapy with steroid-resistant lymphocytes predicting a poor response to steroid therapy.
Interleukins are a group of cytokines produced by activated macrophages and lymphocytes during an immune response. They act on lymphocytes and other leukocytes to stimulate their proliferation and differentiation. A member of this group, interleukin-2 (IL-2) is one of the first cytokines produced by activated T cells and acts in an autocrine manner to stimulate the proliferation and differentiation of the T-cells that produce it. IL-2 produced by activated T lymphocytes in ulcerative colitis may have an important role in perpetuating the mucosal inflammation by activating other inflammatory cells to produce further cytokines, which will produce further inflammatory cell recruitment and activation (Kirman I, et al. J. Dig Dis Sci 1995; 40:291-5). Assuming a patient is responsive to steroid therapy, an important action of steroids is to inhibit IL-2 production to prevent lymphocyte proliferation and associated inflammation (Steroids have multiple actions of which IL-2 inhibition is only one). However, if a patient is steroid-resistant, IL-2 production will not be inhibited by steroids and the inflammatory process will continue uninhibited.

In view of the significant number of patients suffering from autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease for whom steroid treatment is ineffective, there is a need for an effective treatment as an alternative to urgent and invasive surgery.

More specifically the present invention provides in a first aspect a CD25 binding molecule which comprises at least one antigen binding site comprising at least one domain which comprises in sequence, the hypervariable regions CDR1, CDR2 and CDR3; said CDR1 having the amino acid sequence Arg-Tyr-Trp-Met-His, said CDR2 having the amino acid sequence Ala-lie-Tyr-Pro-Gly-Asn-Ser-Asp-Thr-Ser-Tyr-Asn-Gln-Lys-Pho-Glu-Gly, and said CDR3 having the amino acid sequence Asp-Tyr-Gly-Tyr-Tyr-Phe-Asp-Phe; or direct equivalents thereof for use in the treatment of autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease. As shown in studies of examples 1 and 2 below, CD25 binding molecules of the invention modulate Lymphocyte Steroid Sensitivity such that steroid-resistant subjects become steroid-sensitive. Thus, the CD25 binding molecules of the invention may be used in the treatment of patients with steroid-resistant autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease.
Further, the CD25 binding molecules of the invention may also be used in improving the response to steroid therapy in steroid-sensitive patients. Although steroid-sensitive patients would be expected to respond to steroid therapy anyway, the use of the CD25 binding molecules of the invention are predicted to make them respond quicker or may allow a lower dose of steroids to be used, thus significantly reducing the side effects of steroid treatments.

By "CD25 binding molecule" is meant any molecule capable of binding to the CD25 antigen either alone or associated with other molecules to form high affinity IL-2 receptors.

Preferably a CD25 binding molecule is used comprising at least one antigen binding site comprising:
a) a first domain comprising in sequence the hypervariable regions CDR1, CDR2 and CDR3; said CDR1 having the amino acid sequence Arg-Tyr-Trp-Met-His, said CDR2 having the amino acid sequence Ala-Ile-Tyr-Pro-Gly-Asn-Ser-Asp-Thr-Ser-Tyr-Asn-Gin-Lys-Phe-Glu-Gly, and said CDR3 having the amino acid sequence Asp-Tyr-Gly-Tyr-Tyr-Phe-Asp-Phe and,
b) a second domain comprising in sequence the hypervariable regions CDR1', CDR2' and CDR3', said CDR1' having the amino acid sequence Ser-Ala-Ser-Ser-Ser-Ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr;
or direct equivalents thereof.

Unless otherwise indicated, any polypeptide chain is herein described as having an amino acid sequence starting at the N-terminal extremity and ending at the C-terminal extremity.

When the antigen binding site comprises both the first and second domains, these may be located on the same polypeptide molecule or, preferably, each domain may be on a different chain, the first domain being part of an immunoglobulin heavy chain or fragment thereof and the second domain being part of an immunoglobulin light chain or fragment thereof.

The CD25 binding molecule may be one which comprises at least one antigen binding site comprising either a first domain having an amino acid sequence identical or substantially identical to that shown in Seq. Id. No. 1 in EP 449,769B1, starting with amino acid at position 1 and ending with amino acid at position 117 or a first domain as described above and a second domain having an amino acid sequence identical or substantially identical to that shown in Seq. Id. No. 2 in EP 449,769B1, starting with amino acid at position 1 and ending with amino acid at position 104.

A more preferred CD25 binding molecule for use in accordance with the invention is selected from a chimeric anti-CD25 antibody which comprises at least
a) one immunoglobulin heavy chain or fragment thereof which comprises (i) a variable domain comprising in sequence the hypervariable regions CDR1, CDR2 and CDR3 and (ii) the constant part or fragment thereof of a human heavy chain; said CDR1 having the amino acid sequence Arg-Tyr-Trp-Met-His, said CDR2 having the amino acid sequence Ala-Ile-Tyr-Pro-Gly-Asn-Ser-Asp-Thr-Ser-Tyr-Asn-Gln-Lys-Phe-Glu-Gly, and said CDR3 having the amino acid sequence Asp-Tyr-Gly-Tyr-Tyr-Phe-Asp-Phe and
b) one immunoglobulin light chain or fragment thereof which comprises (i) a variable domain comprising in sequence the hypervariable regions CDR1', CDR2' and CDR3' and (ii) the constant part or fragment thereof of a human light chain; said CDR1' having the amino acid sequence Ser-Ala-Ser-Ser-Ser-Ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr; and direct equivalents thereof.

Alternatively, a CD25 binding molecule for use in accordance with the invention may be selected from a single chain binding molecule which comprises an antigen binding site comprising
a) a first domain comprising in sequence the hypervariable regions CDR1, CDR2 and CDR3, said hypervariable regions having the amino acid sequences as shown in Seq. Id. No. 1 in EP 449,769B1,
b) a second domain comprising in sequence the hypervariable regions CDR1', CDR2' and CDR3', said hypervariable regions having the amino acid sequences as shown in Seq. Id. No. 2 in EP 449,769B1, and
c) a peptide linker which is bound either to the N-terminal extremity of the first domain and to the C-terminal extremity of the second domain or to the C-terminal extremity of the first domain and to the N-terminal extremity of second domain; and direct equivalents thereof.

Alternatively, a CD25 binding molecule for use in accordance with the invention may be selected from a single chain binding molecule which comprises an antigen binding site comprising humanized antibodies, e.g. daclizumab which have been disclosed with processes for their preparation in EP 451,216B1.

As it is well known, minor changes in an amino acid sequence such as deletion, addition or substitution of one, more or several amino acids may lead to an allelic form of the original protein which has identical or substantially identical properties, e.g. antigen binding properties. Thus, by the term "direct equivalents thereof" is meant either any single domain CD25 binding molecule (molecule X)
(i) in which the hypervariable regions CDR1, CDR2 and CDR3 taken as a whole are at least 80 % homologous, preferably at least 90 % homologous, more preferably at least 95 % homologous to the hypervariable regions as shown in Seq. Id. No. 1 in EP 449,769B1 or Figure 3 and 4 of EP 451,216B1, and,
(ii) which is capable of inhibiting the binding of Interleukin 2 (IL-2) to its receptor substantially to the same extent as a reference molecule having framework regions identical to those of molecule X but having hypervariable regions CDR1, CDR2 and CDR3 identical to those shown in Seq. Id. No. 1 in EP 449,769B1 or Figure 3 and 4 of EP 451,216B1;
or any CD25 binding molecule having at least two domains per binding site (molecule X')
(i) in which the hypervariable regions CDR1, CDR2, CDR3, CDR1', CDR2' and CDR3' taken as a whole are at least 80 % homologous, preferably at least 90 % homologous, more preferably at least 95 % homologous to the hypervariable regions as shown in Seq. Id. No. 1 and 2 in EP 449,769B1 or Figure 3 and 4 of EP 451,216B1, and
(ii) which is capable of inhibiting the binding of IL-2 to its receptor substantially to the same extent as a reference molecule having framework regions and constant parts identical to molecule X' but having hypervariable regions CDR1, CDR2, CDR3, CDR1', CDR2' and CDR3' identical to those shown in Seq. Id. No. 1 and 2 in EP 449,769B1 or Figure 3 and 4 of EP 451,216B1.

This last criterion may be conveniently tested in various assays as described in EP 449,769B1 or EP 451,216B1.

A more preferred CD25 binding molecule for use in accordance with the invention is a chimeric CD25 antibody, especially a chimeric CD25 antibody comprising at least
a) one heavy chain which comprises a variable domain having an amino acid sequence identical or substantially identical to that shown in Seq. Id. No. 1 in EP 449,769, starting with amino acid at position 1 and ending with amino acid at position 117 and the constant part of a human heavy chain; and
b) one light chain which comprises a variable domain having an amino acid sequence identical or substantially identical to that shown in Seq. Id. No. 2 in EP 449,769B1, starting with glutamic acid at position 1 and ending with glutamic acid at position 104 and the constant part of a human light chain.

The constant part of a human heavy chain may be of the γ1, γ2, γ3, γ4, µ. α1, α2, δ or ε type, preferably of the γ type, more preferably of the γ1 type, whereas the constant part of a human light chain may be of the κ or λ type (which includes the λ1, λ2 and λ3 subtypes) but is preferably of the κ type. The amino acid sequence of all these constant parts are given in Kabat et al., Sequences of Proteins of Immunological Interest, US Department of Health and Human Services, Public Health Service, NIH.

The most preferred CD25 binding molecules according to the invention are the chimeric antibody basiliximab which is commercially available as SIMULECT^{®} from Novartis AG and the humanized antibody daclizumab which is commercially available as ZENAPX^{®} from Roche.
The CD25 binding molecules suitable for use in accordance with the present invention may be produced by techniques disclosed for example in EP 449,769B1 and EP 451,216B1.

As described in EP-B-449,769B1 and EP 451,216B1, the CD25 binding molecules have, on the basis of observed activity in e.g. a *Lymphocyte Steroid Sensitivity* assay, been found to be useful for the prevention and treatment of autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease in a steroid- sensitive or steroid-resistant patient.

The CD25 binding molecule as described above may be used in combination with a further drug substance being effective in the prevention and treatment of autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease. Administration may be concomitant or in sequence.

A pharmaceutical composition used in the methods described herein may comprise a CD25 binding molecule as described above and a pharmaceutically acceptable carrier or diluent therefor.

The CD25 binding molecule as described above may be use in the manufacture of a medicament for use in the methods of treatment or prevention described herein.

A therapeutic combination, e.g. a kit or package, may be used in any of the methods of treatment N prevention as described herein said combination including a pharmaceutical composition comprising a CD25 binding molecule as described above, and further including at least one pharmaceutical composition comprising a further drug substance effective in the prevention and treatment of autoimmune hepatitis, asthma, eczema, vasculitis, temporal arthritis, systemic lupus erythematosis, leukaemia, glomerulonephritis, multiple sclerosis, ulcerative colitis, sarcoid and Crohn's disease.

For the use in accordance with the invention, the appropriate dosage will, of course, vary depending upon, for example, the particular CD25 binding molecule to be employed, the host, the mode of administration and the severity of the condition being treated and the effects desired. Satisfactory results are generally indicated to be obtained at dosages from about 0.1 mg to about 100 mg. Administration may be in a single dose or in several doses over a period of time as long as may be indicated in relation to the time the disease is clinically evident or prophylactically to suppress further clinical relapse, for example a dose from about 5 up to about 100 mg may be administered with a time-lag from 1 day up to five weeks, e.g. every 3 to 6 days, until control or amelioration of the disease is achieved. A preferred dosage regimen comprises administration of 20 mg of CD 25 binding molecule, e.g. basiliximab, on day 0 and administration of a further 20 mg on day 4.The CD25 binding molecule is conveniently administered parenterally, e.g. intravenously, for example, into the antecubital or other peripheral vein. An alternative exemplary dosing regimen is intravenous administration of 40 mg every 28 days until control or amelioration of the disease is achieved.
The intravenous infusions may be prepared as follows: the lyophylized antibodies are mixed together and dispersed into 100 ml sterile buffered saline containing 4.5% wt. of human albumin. This saline dispersion may be administered to the patients either as an intravenous bolus injection or as an intravenous infusion over a 15 minute period.

Investigations so far indicate that the administration of the CD25 binding molecules is free from unacceptable side-effects at the dosage levels employed. Particularly the preferred ones, basiliximab or daclizumab, are safe, approved by the Federal Drug Administration (FDA) of the United States and are commercially available.

Pharmaceutical compositions of the invention may be manufactured in a conventional manner as described, e.g. in EP 449,769B1 or EP 451,216B1.

The CD25 binding molecule may be administered as the sole active ingredient or together with other drugs in immunomodulating regimens or other anti-inflammatory agents. For example, the CD25 binding molecule may be used in accordance with the invention in combination with pharmaceutical compositions effective in various diseases as described above, e.g. with cyclosporins, rapamycins or ascomycins, or their immunosuppressive analogs, e.g. cyclosporin A, cyclosporin G, FK-506, sirolimus, everolimus; corticosteroids e.g. prednisone; cyclophosphamide; azathioprene; methotrexate; gold salts, sulfasalazine, antimalarials, brequinar; leflunomide; mizoribine; mycophenolic acid; mycophenolate mofetil; 15-deoxyspergualine; other immuno-suppressive monoclonal antibodies, e.g. monoclonal antibodies to leukocyte receptors, e.g. MHC, CD2, CD3, CD4, CD7, CD28, B7, CD40, CD45, or CD58 or their ligands; or other immunomodulatory compounds, e.g. CTLA4lg.

If the CD25 binding molecule is co-administered with a further drug substance both may be packaged separately within the same container, with instructions for mixing or concomitant administration. Examples of kits include for example a multi-barrelled syringe or a twin pack containing separate unit dose forms.

The following example illustrates the invention.

### Example 1:

4 male and 4 female subjects are included in the study. The mean age of the study subjects is 52 years (range 34 to 70 years). Two of the subjects are healthy volunteers and six of the subjects are patients with pre-diagnosed ulcerative colitis. Five of the six patients has previously required surgery for poor response to medical therapy and are not taking any anti-inflammatory or immuno-suppressive medication. The sixth subject has quiescent UC (ulcerative colitis) disease and has been taking mesalazine although not on the day of sampling. Therefore, the mesalazine, one action of which is weak inhibition of IL-2, would have been metabolised by the time of sampling. (Basiliximab (trade name SIMULECT^{®} for use *in vitro,* is supplied by Novartis).

*Measurement of lymphocyte steroid sensitivity:* The sensitivity of peripheral blood lymphocytes to glucocorticoids is assessed in a functional assay i.e. an *in vitro* proliferation assay stimulated by phytohaemagglutinin (PHA) and inhibited by dexamethasone. (Hearing SD, et al. Gut 1999;45:382-8; Hearing SD, et al. J Clin Endocrinol Metab 1999;84:4149-54). Peripheral blood samples are collected at 09:00 hours, and peripheral blood mononuclear cells (PBMC) are separated by buoyancy centrifugation. Buoyancy centrifugation includes the separation of lymphocytes from the blood sample by centrifugation over a ficol gradient. Ficol allows red blood cells to fall through, but prevents the through-movement of lymphocytes. Peripheral blood mononuclear cells (lymphocytes and marocyes) are colleted from the ficol/plasma interface. PBMC proliferation is stimulated with PHA and inhibited with dexamethasone at serial 10 fold dilutions between 10⁻⁶ and 10⁻¹⁰ mol/1. i.e. 10⁻⁶, 10⁻⁷, 10⁻⁸, 10⁻⁹, 10⁻¹⁰ mol/1. Cell proliferation is measured by tritiated thymidine uptake i.e. after 2 days of incubation, radioisotape labelled thymidine (³H- thymidine) is added to the lymphocyte culture medium and became incorporated into the DNA of proliferating lymphocyes. After 6 hours the cells are transferred from the incubator and collected on a filter mat using a cell harvester. The cells are then washed out of the well. The lymphocyte DNA that has incorporated the thymidine is trapped in the filter paper. When radioactivity levels are measured the wells which contain high numbers of actively proliferating lymphocytes (and thus will have incorporated lots of thymidine into the DNA) will have high radioactivity levels. Hence radioactivity levels (counts per minute cpm) is used as a measure of lymphocyte proliferation. The results are calculated as mean counts per minute (cpm) of triplicate cultures. Thymidine incorporation for each concentration of dexamethasone is compared to that of PHA stimulated T lymphocytes in the absence of dexamethasone, and expressed as a percentage of the uninhibited positive control. The effect of different concentrations of basiliximab on lymphocyte proliferation is also studied. In addition, an equal concentration of basiliximab is added to each cell culture medium at all concentrations of dexamethasone and the antiproliferative effect measured.
*Effect of SIMULECT® on lymphocyte proliferation:* Serum concentration of 0.2 mg/I of basiliximab produces >90% IL-2R occupancy. To test the antiproliferative *in vitro* effect of basiliximab, various concentrations of between 0.01 and 10 mg/l of basiliximab are incubated with lymphocytes in the absence of steroids. These assays confirm that an *in vitro* concentration of >0.1 to 0.3 mg/l basiliximab is required to produce the *in vitro* effect of basiliximab. For further studies on the same subjects, a concentration of 1 mg/l is used. In total, the same eight subjects are studied in each experiment. The effect of basiliximab at a concentration of 1 mg/l on PHA-induced cell proliferation in the absence of steroids is shown in Table 1

**TABLE 1**

| Subject | 1 | 2 | 3 | 4 | 5 | 6 | 7 | 8 | Mean |
|---|---|---|---|---|---|---|---|---|---|
| PHA alone (cpm) | 38634 | 25228 | 40829 | 32561 | 45441 | 66157 | 28886 | 36990 | 39341 |
| PHA+Basilimab (cpm) | 21827 | 15157 | 27239 | 27538 | 21446 | 35003 | 16122 | 27209 | 23943 |
| Inhibition (%) | 43.5 | 39.9 | 33.3 | 15.4 | 52.8 | 47.1 | 44.2 | 26.4 | 37.8 |

Thus the addition of basiliximab produces a mean of 37.8% inhibition of proliferation in the absence of steroids. However, this level of inhibition is still associated with a poor clinical response in the study of patients with ulcerative colitis (<60%).
*Effect of SIMULECT® on steroid sensitivity:* For all eight subjects, PHA-stimulated lymphocytes are cultured in the presence of increasing concentrations of dexamethasone (10⁻¹⁰ - 10⁻⁶ mol/l) in the presence and absence of basiliximab (1 mg/l). In previous studies of ulcerative colitis carried out by the inventor, maximal suppression (Iₘₐₓ) <60% correlated with a poor response to steroid therapy (clinical steroid resistance). The exact value of Iₘₐₓ cannot be measured as it is the inhibition at an infinitely high level of dexamethasone. Thus, the percentage inhibition of proliferation observed at the maximum dexamethasone concentration applied (10⁻⁶ mol/l) is used as the measure of Iₘₐₓ. In practice, there is good agreement between Iₘₐₓ and the estimated asymptotic value for maximal inhibition obtained from dose-response curves (Hearing SD et al (1999) Clin. Endoctrinol. Metab 84:4149-54). A concentration of dexamethasone of 10⁻⁷ mol/l is approximately equivalent in terms of glucocorticoid activity to the serum concentrations of hydrocortisone achieved with intravenous therapy at standard doses. Thus, to make comparisons with the potential effect of basiliximab *in vivo*, the steroid sensitising effect of basiliximab at this concentration of dexamethasone may also be of interest. The result of the lymphocyte proliferation assay for each subject are summarised in Table 2 which shows the effect of basiliximab on LSS. The subjects are shown in decreasing order of steroid sensitivity (with the most sensitive on the left).

**Table 2**

| | | | | | | | | |
|---|---|---|---|---|---|---|---|---|
| | | | | | | | | |
| PHA alone (cpm) | 38634 | 25229 | 40829 | 32562 | 45442 | 66157 | 28887 | 36991 |
| PHA + Dex 10⁻⁶ (cpm) | 3100 | 4109 | 9477 | 11568 | 21685 | 36495 | 18669 | 24681 |
| PHA + Bas + Dex 10⁻⁶ | 639 | 4533 | 3059 | 3801 | 3563 | 6910 | 3352 | 5924 |
| Iₘₐₓ (Dex)(%) | 92 | 84 | 77 | 64 | 52 | 45 | 35 | 33 |
| Iₘₐₓ (Dex + Bas) (%) | 98 | 82 | 93 | 88 | 92 | 90 | 88 | 84 |
| % suppression Dex 10⁻⁷ | 87 | 79 | 65 | 42 | 20 | 32 | 29 | 36 |
| % suppression Dex 10⁻⁷+Bas | 98 | 84 | 88 | 83 | 87 | 83 | 86 | 81 |
| Steroid-sensitive (SS) or Steroid-resistant (SR) | SS | SS | SS | SS | SR | SR | SR | SR |

Four subjects are steroid-sensitive (Iₘₐₓ >60%; subjects 1-4) and four are steroid-resistant (Iₘₐₓ <60%; subjects 5-8). It can be seen from the effect on Iₘₐₓ that for all steroid-resistant subjects, LSS is modulated by basiliximab and all become Steroid-sensitive. Furthermore, the effect on the % suppression of lymphocytes at a concentration of 10⁻⁷ mol/l dexamethasone can also be seen.
The modulation of lymphocyte steroid sensitivity by basiliximab is equally applicable to alter the steroid sensitivity of steroid-resistant patients with autoimmune hepatitis, asthma, eczema, vasculitis, temporal arthritis, systemic lupus erythematosis, leukaemia, glomerulonephritis, multiple sclerosis, sarcoid and Crohn's disease, as in all of these conditions, a poor response to steroid therapy is likely to be associated with steroid-resistant lymphocytes. Modulation of lymphocyte steroid sensitivity by basiliximab to make steroid-resistant lymphocytes Steroid-sensitive would allow patients previously non-responsive to steroid therapy to be successfully treated using steroids. In addition, Steroid-sensitive patients, who would be expected to respond to steroid therapy, would respond more quicky to steroid therapy if treated with basiliximab.

### Example 2:

Peripheral blood lymphocytes are isolated from 32 subjects (25 healthy volunteers and 7 patients with quiescent Ulcerative Colitis), on 41 occasions, using buoyant density centrifugation. Lymphocyte steroid sensitivity is assessed by measuring the antiproliferative effect of dexamethasone (DEX) on phytohaemagglutinin stimulated lymphocytes. Maximum steroid induced suppression is expressed as a % of control lymphocyte proliferation (Iₘₐₓ). Iₘₐₓ is measured in the absence and presence of basiliximab (1 mg/L).
The addition of basiliximab significantly enhanced the anti-proliferative effect of DEX (Wilcoxon signed-rank test, p<0.0001).

| | Median Iₘₐₓ (%) | Interquartile range |
|---|---|---|
| Dexamethasone | 78.0 | 66.1-86.4 |
| Dexamethasone + Basiliximab | 92.3 | 89.3-95.6 |

All 8 steroid-resistant subjects (Iₘₐₓ <60%) are modulated (*in vitro*) to steroid-sensitive by the addition of basiliximab. Thus, basiliximab has a marked *in vitro* steroid enhancing effect.

## Claims

1. A CD25 binding molecule for use in the prevention or treatment of autoimmune hepatitis, asthma, eczema, vasculitis, temporal arteritis, systemic lupus erythematosis, leukaemia, glomerulonephritis, multiple sclerosis, ulcerative colitis, sarcoid or Crohn's disease in a steroid-resistant patient; wherein said CD25 binding molecule comprises at least one antigen binding site comprising:
(a) at least one domain which comprises in sequence, the hypervariable regions CDR1, CDR2 and CDR3; said CDR1 having the amino acid sequence Arg-Tyr-Trp-Met-His, said CDR2 having the amino acid sequence Ala-Ile-Tyr-Pro-Gly-Asn-Ser-Asp-Thr-Ser-Tyr-Asn-Gln-Lys-Phe-Glu-Gly, and said CDR3 having the amino acid sequence Asp-Tyr-Gly-Tyr-Tyr-Phe-Asp-Phe;
(b) and optionally a further domain comprising in sequence the hypervariable regions CDR1', CDR2' and CDR3', said CDR1' having the amino acid sequence Ser-Ala-Ser-Ser-Ser-ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr;
or direct equivalents thereof,
wherein the direct equivalent is a single domain CD25 binding molecule (molecule X) (i) in which the hypervariable regions CDR1, CDR2 and CDR3 taken as a whole are at least 80 % homologous to CDR1, CDR2 and CDR3 amino acid sequences as defined in (a), and (ii) which is capable of inhibiting the binding of Interleukin 2 to its receptor substantially to the same extent as a reference molecule having framework regions identical to those of molecule X but having hypervariable regions CDR1, CDR2 and CDR3 identical to CDR1, CDR2 and CDR3 amino acid sequences as defined in (a);
or the direct equivalent is equivalent is a CD25 binding molecule having at least two domains per binding site (molecule X') (i) in which the hypervariable regions CDR1, CDR2 and CDR3, taken as a whole are at least 80 % homologous to CDR1, CDR2 and CDR3 having the amino acid sequences as defined in (a) and the hypervariable regions CDR1', CDR2' and CDR3', taken as a whole are at least 80 % homologous to CDR1', CDR2' and CDR3' amino acid sequences as defined in (b), and (ii) which is capable of inhibiting the binding of Interleukin 2 to its receptor substantially to the same extent as a reference molecule having framework regions identical to those of molecule X' but having hypervariable regions CDR1, CDR2 and CDR3 identical to CDR1, CDR2 and CDR3 amino acid sequences as defined in (a) and hypervariable regions CDR1', CDR2' and CDR3' identical to CDR1', CDR2' and CDR3' amino acid sequences as defined in (b).

2. The CD25 binding molecule for use as claimed in claim 1 wherein the CD25 binding molecule is basiliximab or daclizumab.

3. The CD25 binding molecule for use as claimed in claim 1 wherein the hypervariable regions CDR1, CDR2 and CDR3 taken as a whole are at least 90 %, preferably at least 95% homologous to CDR1, CDR2 and CDR3 amino acid sequences as defined in claim 1.

4. The CD25 binding molecule for use as claimed in claim 1, wherein the CD25 binding molecule comprises at least one antigen binding site comprising: a) a first domain comprising in sequence the hypervariable regions CDR1, CDR2 and CDR3 as defined in claim 1 and, b) a second domain comprising in sequence the hypervariable regions CDR1', CDR2' and CDR3', said CDR1' having the amino acid sequence Ser-Ala-Ser-Ser-Ser-ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr.

5. The CD25 binding molecule for use as claimed in claim 4 wherein the hypervariable regions CDR1, CDR2 and CDR3 taken as a whole are at least 90 %, preferably at least 95% homologous to CDR1, CDR2 and CDR3 amino acid sequences as defined in claim 1, and the hypervariable regions CDR1', CDR2' and CDR3', taken as a whole are at least 90 %, preferably at least 95% homologous to CDR1', CDR2' and CDR3' amino acid sequences as defined in claim 4.

6. The CD25 binding molecule for use as claimed in claim 1, wherein the CD25 binding molecule is a chimeric anti-CD25 antibody which comprises at least
a) one immunoglobulin heavy chain or fragment thereof which comprises (i) a variable domain comprising in sequence the hypervariable regions CDR1, CDR2 and CDR3 as defined in claim 1 and (ii) the constant part or fragment thereof of a human heavy chain; and
b) one immunoglobulin light chain or fragment thereof which comprises (i) a variable domain comprising in sequence the hypervariable regions CDR1', CDR2' and CDR3'and (ii) the constant part or fragment thereof of a human light chain; said CDR1' having the amino acid sequence Ser-Ala-Ser-Ser-Ser-ile-Ser-Tyr-Met-Gln, said CDR2' having the amino acid sequence Asp-Thr-Ser-Lys-Leu-Ala-Ser, and said CDR3' having the amino acid sequence His-Gln-Arg-Ser-Ser-Tyr-Thr; or a direct equivalent thereof.

7. The CD25 binding molecule for use as claimed in claim 1, wherein the CD25 binding molecule is used in combination with a steroid or a further drug substance selected from:
cyclosporins, rapamycins or ascomycin, or their immunosuppressive analogs, e.g. cyclosporin A, cyclosporin G, FK-506, sirolimus, everolimus;
corticosteroids, e. g. prednisone;
cyclophosphamide; azathioprene; methotrexate; gold salts; sulfasalazine; antimalarials; brequinar; leflunomide; mizoribine; mycophenolic acid; mycophenolate mofetil; 15-deoxyspergualine;
suppressive monoclonal antibodies, e. g. monoclonal antibodies to leukocyte receptors, e. g. MHC, CD2,CD3, CD4, CD7, CD28, B7, CD40, CD45, or CD58 or their ligands;
other immunomodulatory compounds, e. g. CTLA41g.

8. A pharmaceutical composition for the prevention or treatment of autoimmune hepatitis, asthma, eczema, vasculitis, temporal arteritis, systemic lupus erythematosis, leukaemia, glomerulonephritis, multiple sclerosis, ulcerative colitis, sarcoid or Crohn's disease in a steroid-resistant patient; comprising a CD25 binding molecule as defined in any one of claims 1-7and a pharmaceutically acceptable carrier or diluent therefor.

9. Use of a CD25 binding molecule as defined in any one of claims 1 to 7 for the preparation of a medicament for the prevention or treatment of autoimmune hepatitis, asthma, eczema, vasculitis, temporal arteritis, systemic lupus erythematosis, leukaemia, glomerulonephritis, multiple sclerosis, ulcerative colitis, sarcoid or Crohn's disease in a steroid-resistant patient.

10. A therapeutic combination comprising (a) a CD25 binding molecule as defined in any one of claims 1 to 7 and (b) a steroid, for use in a method for the prevention or treatment of autoimmune hepatitis, asthma, eczema, vasculitis, temporal arteritis, systemic lupus erythematosis, leukaemia, glomerulonephritis, multiple sclerosis, ulcerative colitis, sarcoid or Crohn's disease in a steroid-resistant patient.

11. The therapeutic combination for use as claimed in claim 10, wherein the CD25 binding molecule is basiliximab and said use is in the treatment of ulcerative colitis.

## Patentansprüche

1. CD25-Bindemolekül zur Verwendung zum Vorbeugen oder Behandeln von Autoimmunhepatitis, Asthma, Ekzema, Vaskulitis, temporärer Arthritis, systemischer Lupus-Erythematose, Leukämie, Glomerulonephritis, multipler Sklerose, Colitis ulcerosa, Sarcoidose oder Morbus Crohn in einem steroidresistenten Patienten, wobei das CD25-Bindemolekül mindestens eine Antigen-Bindestelle enthält, umfassend:
(a) mindestens eine Domäne, die in Reihe die hypervariablen Regionen CDR1, CDR2 und CDR3 aufweist, wobei CDR1 die Aminosäuresequenz Arg-Tyr-Trp-Met-His besitzt, CDR2 die Aminosäuresequenz Ala-Ile-Tyr-Pro-Gly-Asn-Ser-Asp-Thr-Ser-Tyr-Asn-Gln-Lys-Phe-Glu-Gly und CDR3 die Aminosäuresequenz Asp-Tyr-Gly-Tyr-Tyr-Phe-Asp-Phe;
(b) und gegebenenfalls eine weitere Domäne, die in Reihe die hypervariablen Regionen CDR1', CDR2' und CDR3' enthält, wobei CDR1' die Aminosäuresequenz Ser-Ala-Ser-Ser-Ser-Ile-Ser-Tyr-Met-Gln besitzt, CDR2' die Aminosäuresequenz Asp-Thr-Ser-Lys-Leu-Ala-Ser und CDR3' die Aminosäuresequenz His-Gln-Arg-Ser-Ser-Tyr-Thr;
oder direkte Äquivalente davon,
wobei das direkte Äquivalent ein CD25-Bindemolekül mit einer einzelnen Domäne (Molekül X) ist, worin (i) die hypervariablen Regionen CDR1, CDR2 und CDR3 insgesamt zu mindestens 80% homolog sind zu den Aminosäuresequenzen von CDR1, CDR2 und CDR3, wie in (a) definiert, und (ii) das die Bindung von Interleukin-2 an seinen Rezeptor im Wesentlichen genauso stark wie ein Referenzmolekül zu hemmen vermag, dessen Gerüststrukturbereiche identisch sind zu denen von Molekül X, das aber hypervariable Regionen CDR1, CDR2 und CDR3 enthält, die identisch sind zu den Aminosäuresequenzen von CDR1, CDR2 und CDR3, wie in (a) definiert;
oder das direkte Äquivalent ist ein CD25-Bindemolekül mit mindestens zwei Domänen pro Bindungsstelle (Molekül X'), (i) worin die hypervariablen Regionen CDR1, CDR2 und CDR3 insgesamt zu mindestens 80% homolog sind zu CDR1, CDR2 und CDR3 mit den Aminosäuresequenzen, wie in (a) definiert, und die hypervariablen Regionen CDR', CDR2' und CDR3' insgesamt zu mindestens 80% homolog sind zu den Aminosäuresequenzen von CDR1', CDR2' und CDR3', wie in (b) definiert, und (ii) das die Bindung von Interleukin-2 an seinen Rezeptor im Wesentlichen genauso stark wie ein Referenzmolekül zu hemmen vermag, dessen Gerüststrukturbereiche identisch sind zu denen von Molekül X', aber identische hypervariable Regionen CDR1, CDR2 und CDR3 hat zu den Aminosäuresequenzen von CDR1, CDR2 und CDR3, wie in (a) definiert, und identische hypervariable Regionen CDR1', CDR2' und CDR3' zu den Aminosäuresequenzen von CDR1', CDR2' und CDR3', wie in (b) definiert.

2. CD25-Bindemolekül zur Verwendung nach Anspruch 1, wobei das CD25-Bindemolekül Basiliximab oder Daclizumab ist.

3. CD25-Bindemolekül zur Verwendung nach Anspruch 1, wobei die hypervariablen Regionen CDR1, CDR2 und CDR3 insgesamt zu mindestens 90%, vorzugsweise zu mindestens 95% homolog sind zu den Aminosäuresequenzen von CDR1, CDR2 und CDR3 nach Anspruch 1.

4. CD25-Bindemolekül zur Verwendung nach Anspruch 1, wobei das CD25-Bindemolekül umfasst: mindestens eine Antigenbindungsstelle mit a) einer ersten Domäne, die in Reihe die hypervariablen Regionen CDR1, CDR2 und CDR3 nach Anspruch 1 umfasst und b) einer zweiten Domäne, die in Reihe die hypervariablen Regionen CDR1', CDR2' und CDR3' umfasst, wobei CDR1' die Aminosäuresequenz Ser-Ala-Ser-Ser-Ser-Ile-Ser-Tyr-Met-Gln hat, CDR2' die Aminosäuresequenz Asp-Thr-Ser-Lys-Leu-Ala-Ser und CDR3' die Aminosäuresequenz His-Gln-Arg-Ser-Ser-Tyr-Thr.

5. CD25-Bindemolekül zur Verwendung nach Anspruch 4, wobei die hypervariablen Regionen CDR1, CDR2 und CDR3 insgesamt zu mindestens 90%, vorzugsweise zu mindestens 95% homolog sind zu den Aminosäuresequenzen von CDR1, CDR2 und CDR3 nach Anspruch 1 und die hypervariablen Regionen CDR1', CDR2' und CDR3' insgesamt zu mindestens 90%, vorzugsweise zu mindestens 95% homolog sind zu den Aminosäuresequenzen von CDR1', CDR2' und CDR3' nach Anspruch 4.

6. CD25-Bindemolekül zur Verwendung nach Anspruch 1, wobei das CD25-Bindemolekül ein chimärer Anti-CD25-Antikörper ist, der mindestens umfasst:
a) eine schwere Immunglobulinkette oder ein Fragment davon, umfassend (i) eine variable Domäne, die in Reihe die hypervariablen Regionen CDR1, CDR2 und CDR3 nach Anspruch 1 enthält, und (ii) den konstanten Bereich oder ein Fragment davon von der schweren Kette aus Mensch; und
b) eine leichte Immunglobulinkette oder ein Fragment davon, umfassend (i) eine variable Domäne, die in Reihe die hypervariablen Regionen CDR1', CDR2' und CDR3' enthält und (ii) den konstanten Teil oder ein Fragment davon einer leichten Kette aus Mensch, wobei CDR1' die Aminosäuresequenz Ser-Ala-Ser-Ser-Ser-Ile-Ser-Tyr-Met-Gln besitzt, CDR2' die Aminosäuresequenz Asp-Thr-Ser-Lys-Leu-Ala-Ser, und CDR3' die Aminosäuresequenz His-Gln-Arg-Ser-Ser-Tyr-Thr oder ein direktes Äquivalent davon.

7. CD25-Bindemolekül zur Verwendung in Anspruch 1, wobei das CD25-Bindemolekül zusammen mit einem Steroid oder einem weiteren Wirkstoff verwendet wird, ausgewählt aus:
Cyclosporinen, Rapamycinen oder Ascomycin oder ihren immunsuppressiven Analoga, beispielsweise Cyclosporin-A, Cyclosporin-G, FK-506, Sirolimus, Everolimus;
Corticosteroiden, beispielsweise Prednison;
Cyclophosphamid; Azathiopren; Methotrexat; Goldsalzen; Sulfasalazin; Antimalariamittel; Brequinar; Leflunomid; Mizoribin; Mycophenolsäure; Mycophenolat-Mofetil; 15-Desoxyspergualin;
suppressiven monoklonalen Antikörpern, beispielsweise monoklonalen Antikörpern gegen Leukozytenrezeptoren, beispielsweise MHC, CD2, CD3, CD4, CD7, CD28, B7, CD40, CD45 oder CD58 oder ihren Liganden;
anderen immunmodulatorischen Verbindungen, beispielsweise CTLA41 g.

8. Pharmazeutische Zusammensetzung zur Vorbeugung oder Behandlung von Autoimmunhepatitis, Asthma, Ekzem, Vaskulitis, temporärer Arthritis, systemischer Lupus-Erythematose, Leukämie, Glomerulonephritis, multipler Sklerose, Colitis ulcerosa, Sarcoidose oder Morbus Crohn in einem steroidresistenten Patienten, umfassend ein CD25-Bindemolekül nach einem der Ansprüche 1 bis 7 und einen pharmazeutisch verträglichen Träger oder ein Verdünnungsmittel hierfür.

9. Verwendung eines CD25-Bindemoleküls nach einem der Ansprüche 1 bis 7 zur Herstellung eines Medikaments zur Vorbeugung oder Behandlung von Autoimmunhepatitis, Asthma, Ekzem, Vaskulitis, temporärer Arthritis, systemischer Lupus-Erythematose, Leukämie, Glomerulonephritis, multipler Sklerose, Colitis ulcerosa, Sarcoidose oder Morbus Crohn in einem steroidresistenten Patienten.

10. Therapeutische Kombination, umfassend (a) ein CD25-Bindemolekül nach einem der Ansprüche 1 bis 7 und (b) ein Steroid zur Verwendung in einem Verfahren zur Vorbeugung oder Behandlung von Autoimmunhepatitis, Asthma, Ekzem, Vaskulitis, temporärer Arthritis, systemischer Lupus-Erythematose, Leukämie, Glomerulonephritis, multipler Sklerose, Colitis ulcerosa, Sarcoidose oder Morbus Crohn in einem steroidresistenten Patienten.

11. Therapeutische Kombination zur Verwendung nach Anspruch 10, wobei das CD25-Bindemolekül Basiliximab ist, und die Verwendung die Behandlung von Colitis ulcerosa.

## Revendications

1. Molécule de fixation au CD25 à utiliser dans la prévention ou le traitement de l'hépatite auto-immune, de l'asthme, de l'eczéma, de la vascularite, de l'artérite temporale, du lupus érythémateux aigu disséminé, de la leucémie, de la glomérulonéphrite, de la sclérose en plaques, de la rectocolite hémorragique, de la sarcoïde ou de la maladie de Crohn chez un patient résistant aux stéroïdes ; la molécule de fixation au CD25 comprenant au moins un site de fixation d'antigène comprenant :
(a) au moins un domaine qui comprend en succession les régions CDR1, CDR2 et CDR3 hypervariables ; CDR1 ayant la séquence d'acides aminés Arg-Tyr-Trp-Met-His, CDR2 ayant la séquence d'acides aminés Ala-Ile-Tyr-Pro-Gly-Asn-Ser-Asp-Thr-Ser-Tyr-Asn-Gln-Lys-Phe-Glu-Gly et CDR3 ayant la séquence d'acides aminés Asp-Tyr-Gly-Tyr-Tyr-Phe-Asp-Phe ;
(b) et éventuellement un autre domaine comprenant en succession les régions CDR1', CDR2' et CDR3' hypervariables, CDR1' ayant la séquence d'acides aminés Ser-Ala-Ser-Ser-Ser-ile-Ser-Tyr-Met-Gln, CDR2' ayant la séquence d'acides aminés Asp-Thr-Ser-Lys-Leu-Ala-Ser et CDR3' ayant la séquence d'acides aminés His-Gln-Arg-Ser-Ser-Tyr-Thr ;
ou ses équivalents directs,
dans laquelle l'équivalent direct est une molécule de fixation au CD25 à domaine unique (molécule X) (i) dans laquelle les régions CDR1, CDR2 et CDR3 hypervariables prises dans leur ensemble sont homologues à au moins 80 % des séquences d'acides aminés CDR1, CDR2 et CDR3 telles que définies dans (a) et (ii) qui est apte à inhiber la fixation de l'interleukine 2 à son récepteur sensiblement dans la même mesure qu'une molécule témoin ayant des régions de squelette identiques à celles de la molécule X mais ayant des régions CDR1, CDR2 et CDR3 hypervariables identiques aux séquences d'acides aminés CDR1, CDR2 et CDR3 telles que définies dans (a) ;
ou l'équivalent direct est une molécule de fixation au CD25 ayant au moins deux domaines par type de fixation (molécule X') (i) dans laquelle les régions CDR1, CDR2 et CD3 hypervariables prises dans leur ensemble sont homologues à au moins 80 % de CDR1, CDR2 et CDR3 ayant les séquences d'acides aminés telles que définies par (a) et les régions CDR1', CDR2' et CDR3' hypervariables prises dans leur ensemble sont homologues à au moins 80 % des séquences d'acides aminés CDR1', CDR2' et CDR3' telles que définies dans (b) et (ii) qui est apte à inhiber la fixation de l'interleukine 2 à son récepteur sensiblement dans la même mesure qu'une molécule témoin ayant des régions de squelette identiques à celles de la molécule X' mais ayant des régions CDR1, CDR2 et CDR3 hypervariables identiques aux séquences d'acides aminés CDR1, CDR2 et CDR3 telles que définies dans (a) et des régions CDR1', CDR2' et CDR3' hypervariables identiques aux séquences d'acides aminés CDR1', CDR2' et CDR3' telles que définies dans (b).

2. Molécule de fixation au CD25 à utiliser comme revendiqué à la revendication 1, dans laquelle la molécule de fixation au CD25 est le basiliximab ou le daclizumab.

3. Molécule de fixation au CD25 à utiliser comme revendiqué à la revendication 1, dans laquelle les régions CFR1, CDR2 et CDR3 hypervariables prises dans leur ensemble sont homologues à au moins 90 % et, de préférence, à au moins 95 % aux séquences d'acides aminés CDR1, CDR2 et CDR3 telles que définies à la revendication 1.

4. Molécule de fixation au CD25 à utiliser comme revendiqué dans la revendication 1, dans laquelle la molécule de fixation au CD25 comprend au moins un site de fixation d'un antigène comprenant : a) un premier domaine comprenant en succession les régions CDR1, CDR2 et CDR3 hypervariables telles que définies à la revendication 1) et b) un deuxième domaine comprenant en succession les CDR1', CDR2' et CDR3' hypervariables, CDR1' ayant la séquence d'acides aminés Ser-Ala-Ser-Ser-Ser-ile-Ser-Tyr-Met-Gln, CDR2' ayant la séquence d'acides aminés Asp-Thr-Ser-Lys-Leu-Ala-Ser et CDR3' ayant la séquence d'acides aminés His-Gln-Arg-Ser-Ser-Tyr-Thr.

5. Molécule de fixation au CD25 à utiliser comme revendiqué à la revendication 4, dans laquelle les régions CDR1, CDR2 et CDR3 hypervariables prises dans leur ensemble sont homologues au moins à 90 %, de préférence au moins à 95 %, des séquences d'acides aminés CDR1, CDR2 et CDR3 telles que définies à la revendication 1 et les régions CDR1', CDR2' et CDR3' hypervariables prises dans leur ensemble sont homologues au moins à 90 % et, de préférence, au moins à 95 % des séquences d'acides aminés CDR1', CDR2' et CDR3' telles que définies à la revendication 4.

6. Molécule de fixation au CD25 à utiliser comme revendiqué à la revendication 1, dans laquelle la molécule de fixation au CD25 est un anticorps chimère anti-CD25 qui comprend au moins
a) une chaîne d'immunoglobuline lourde ou l'un de ses fragments qui comprend (i) un domaine variable comprenant en succession les régions CDR1, CDR2 et CDR3 hypervariables telles que définies à la revendication 1 et (ii) la partie constante ou son fragment d'une chaîne humaine lourde ; et
b) une chaîne d'immunoglobuline légère ou son fragment qui comprend (i) un domaine variable comprenant en succession les régions CDR1', CDR2' et CDR3' hypervariables et (ii) la partie constante ou son fragment d'une chaîne humaine légère ; le CDR1' ayant la séquence d'acides aminés Ser-Ala-Ser-Ser-Ser-ile-Ser-Tyr-Met-Gln, le CDR2' ayant la séquence d'acides aminés Asp-Thr-Ser-Lys-Leu-Ala-Ser et le CDR3' ayant la séquence d'acides aminés His-Gln-Arg-Ser-Ser-Tyr-Thr ; ou son équivalent direct.

7. Molécule de fixation au CD25 à utiliser comme revendiqué à la revendication 1, dans laquelle la molécule de fixation au CD25 est utilisée en combinaison avec un stéroïde ou une autre substance médicamenteuse choisis parmi :
les cyclosporines, les rapamycines ou l'ascomycine ou leurs analogues immunosuppresseurs, par exemple la cyclosporine A, la cyclosporine G, FK-506, le sirolimus, l'éverolimus ;
les corticostéroïdes, par exemple la prednisone ;
le cyclophosphamide ; l'azothioprène ; le méthotrexate ; les sels d'or ; la surfasalazine ; les antipaludiques ; le bréquinar ; le léflunomide ; la mizoribine ; l'acide mycophénolique ; le mycophénolate mofétil ; la 15-désoxyspergualine ;
les anticorps monoclonaux suppresseurs, par exemple les anticorps monoclonaux aux récepteurs de leucocytes, par exemple MHC, CD2, CD3, CD4, CD7, CD28, B7, CD40, CD45 ou CD58 ou leurs ligands ;
d'autres composés immuno-modulateurs, par exemple CTLA41g.

8. Composition pharmaceutique de prévention ou de traitement de l'hépatite auto-immune, de l'asthme, de l'eczéma, de la vascularite, de l'artérite temporale, du lupus érythémateux aigu disséminé, de la leucémie, de la glomérulonéphrite, de la sclérose en plaques, de la rectocolite hémorragique, de la sarcoïde ou de la maladie de Crohne chez un patient résistant aux stéroïdes ; comprenant une molécule de fixation au CD25 telle que définie à l'une quelconque des revendications 1 à 7 et un véhicule ou diluant acceptable pharmaceutiquement pour celle-ci.

9. Utilisation d'une molécule de fixation au CD25 telle que définie à l'une quelconque des revendications 1 à 7 pour la préparation d'un médicament de prévention ou de traitement de l'hépatite auto-immune, de l'asthme, de l'eczéma, de la vascularite, de l'artérite temporale, du lupus érythémateux aigu disséminé, de la leucémie, de la glomérulonéphrite, de la sclérose en plaques, de la rectocolite hémorragique, de la sarcoïde ou de la maladie de Crohne chez un patient résistant aux stéroïdes.

10. Combinaison thérapeutique comprenant (a) une molécule de fixation au CD25 telle que définie à l'une quelconque des revendications 1 à 7 et (b) un stéroïde à utiliser dans un procédé de prévention ou de traitement de l'hépatite auto-immune, de l'asthme, de l'eczéma, de la vascularite, de l'artérite temporale, du lupus érythémateux aigu disséminé, de la leucémie, de la glomérulonéphrite, de la sclérose en plaques, de la rectocolite hémorragique, de la sarcoïde ou de la maladie de Crohne chez un patient résistant aux stéroïdes.

11. Combinaison thérapeutique à utiliser telle que revendiquée à la revendication 10, dans laquelle la molécule de fixation au CD25 est le basiliximab et l'utilisation est le traitement de la rectocolite hémorragique.
